# EUROPEAN PATENT SPECIFICATION

(11) **EP 0 941 100 B1**
(45) Date of publication and mention of the grant of the patent: **28.05.2003**
(21) Application number: 97950036.0
(22) Date of filing: 29.10.1997
(51) Int. Cl.: A61K 31/70, A61P 31/12, A61P 31/18, A61P 31/22

(54) **PHARMACEUTICAL COMPOSITIONS CONTAINING LAMIVUDINE AND ZIDOVUDINE**
PHARMAZEUTISCHE ZUBEREITUNGEN, WELCHE ZIDOVUDIN UND LAMIVUDINE ENTHALTEN
COMPOSITIONS PHARMACEUTIQUES CONTENANT DE LA LAMIVUDINE ET DE LA ZIDOVUDINE

(30) Priority: 31.10.1996 GB 9622681
(43) Date of publication of application: 15.09.1999
(73) Proprietor: GLAXO GROUP LIMITED, Greenford, Middlesex UB6 ONN (GB)
(72) Inventor: GOODSON, Gary, Wayne, Research Triangle Park, NC 27709 (US); WOOD, Allen, Wayne, Research Triangle Park, NC 27709 (US); FORD, Katherine, Jeannette, Research Triangle Park, NC 27709 (US)
(74) Representative: Quillin, Helen Kaye
(86) International application number: EP9705953
(87) International publication number: WO98018477

(56) References cited:
- WO-A-92/20344
- MATHEZ D ET AL: "INFECTIOUS AMPLIFICATION OF WILD-TYPE HUMAN IMMUNODEFICIENCY VIRUS FROM PATIENTS' LYMPHOCYTES AND MODULATION BY REVERSE TRANSCRIPTASE INHIBITORS IN VITRO" ANTIMICROBIAL AGENTS AND CHEMOTHERAPY, vol. 37, no. 10, 1 October 1993, pages 2206-2211, XP000578786
- SUCKER H.; FUCHS P.; SPEISER P. : " PHARMAZEUTISCHE TECHNOLOGIE. " 1991 , STUTTGART, THIEME VERLAG.; DE XP002060945 see page 259, column 1, paragraph 4 - page 260, column 1, paragraph 2

## Description

The present Invention relates to novel pharmaceutical compositions combining the agents lamivudine and zidovudine into a single dosage form, useful in the treatment of diseases in mammals, including humans.

The present Invention relates to novel pharmaceutical compositions combining the agents lamivudine and zidovudine into a single dosage form, useful in the treatment of diseases in mammals, including humans. The compositions of the present Invention are particularly useful for treating viral infections, particularly retroviral infections, including human immunodeficiency virus (HIV).

HIV causes a variety of clinical conditions including acquired immune deficiency syndrome (AIDS) and chronic neurological disorders. The recent advent of a variety of multiple-drug treatment regimens has dramatically improved the treatment of HIV infected patients. Prior to these multiple-drug regimens, treatment was often limited to single drugs with limited effectiveness.

Single drug treatment regimens typically require long term treatment increasing the incidence of unwanted side effects. Moreover, single drug therapies are particularly vulnerable to mutations in the HIV virus, leading to drug resistant variants of HIV.

The use of multiple drug therapies may reduce the development of drug-resistant strains of HIV because one drug will usually cancel out mutations against other drugs. Multiple-drug therapies may even inhibit replication of HIV viruses for a period of time sufficient to eliminate HIV from the body.

The success of modern multiple-drug treatments for HIV often requires strict compliance with a complex treatment regimen that can require the administration of many different drugs per day, administered at precisely timed intervals with careful attention to diet. Patient non-compliance is a well known problem accompanying such complex treatment regimens. See Goodman & Gilman, The Pharmacological Basis of Therapeutics, 9th ed., pp. 1704-1705 (1996). Patient non-compliance is an important problem in the treatment of HIV because such noncompliance may lead to the emergence of multiple-drug resistant strains of HIV.

Two of the many compounds which are commonly included in multiple-drug treatment regimens for HIV are zidovudine and lamivudine. Lamivudine (also known as 3TC™) is a synthetic nucleoside analogue, chemically known as (2R,cis)-4-amino-1 -(2-hydroxymethyl-1 ,3-oxathiolan-5-yl)-(1 H)-pyrimidin-2-one. Lamivudine has also been referred to as (-)-2',3'-dideoxy, 3'thyacytidine. Lamivudine has proven antiviral activity against human immunodeficiency virus (HIV) and other viruses such as hepatitis B. Lamivudine is commercially available from Glaxo Wellcome Inc. under the tradename EPIVIR™. Lamivudine and its use against HIV are described in EP 0382526 and WO91/17159. Crystalline forms of lamivudine are described in WO92/21676. Combinations of lamivudine with other reverse transcriptase inhibitors, in particular zidovudine, are described in WO92/20344.

Zidovudine, chemically known as 3'-azido-3'-deoxythymidine, is a pyrimidine nucleoside analogue commercially available from Glaxo Wellcome Inc. under the tradename RETROVIR™ for the treatment of HIV and other viruses. Zidovudine is further described in United States Patent Nos. 4,818,538, 4,828,838, 4,724,232, 4,833,130 and 4,837,208.

In November of 1995, the FDA granted accelerated approval for the use of lamivudine in combination with zidovudine for first-line treatment of HIV-infection in adults and children. Lamivudine exhibits unexpected advantages when used in combination with known inhibitors of HIV replication. In particular, lamivudine shows a synergistic antiviral effect when used in combination with zidovudine. In controlled clinical trials, combination therapy with lamivudine and zidovudine delayed the emergence of zidovudine-resistant mutations of HIV.

Segregation of active ingredients in pharmaceutical powders and granulations is a widely recognized problem that can result in inconsistent dispersions of the active ingredients in final dosage forms. Some of the main factors contributing to segregation are particle size, shape and density. Segregation is particularly troublesome when attempting to formulate a single homogenous tablet containing multiple active ingredients having different densities and different particle sizes. Previous attempts to formulate tablets containing lamivudine and zidovudine were hindered by precisely such segregation problems. Although mixed blends were initially homogeneous, the active ingredients segregated during material handling and prior to tablet compression.

Glidants are substances that have traditionally been used to improve the flow characteristics of granulations and powders by reducing interparticulate friction. See Lieberman, Lachman, & Schwartz, Pharmaceutical Dosage Forms: Tablets, Volume 1, p. 177-178 (1989). Glidants are typically added to pharmaceutical compositions immediately prior to tablet compression to facilitate the flow of granular material into the die cavities of tablet presses. Glidants include: colloidal silicon dioxide, asbestos free talc, sodium aluminosilicate, calcium silicate, powdered cellulose, microcrystalline cellulose, corn starch, sodium benzoate, calcium carbonate, magnesium carbonate, metallic stearates, calcium stearate, magnesium stearate, zinc stearate, stearowet C, starch, starch 1500, magnesium lauryl sulfate, and magnesium oxide.

Research into the problem of segregation in pharmaceutical compositions has surprisingly demonstrated that glidants can be used to increase and aid blend composition homogeneity. The novel compositions of the present Invention use glidants to effect and maintain homogeneity of active ingredients during handling prior to tablet compression.

It is therefore an object of the present Invention to provide a pharmaceutical formulation combining the active ingredients lamivudine and zidovudine, or pharmaceutically acceptable derivatives thereof, in a sufficiently homogenized form.

A further object of the present Invention is to utilize glidants to reduce the segregation of active ingredients in pharmaceutical compositions during precompression material handling.

A still further object of the present Invention is to provide a pharmaceutical formulation combining the active ingredients lamivudine and zidovudine, or pharmaceutically acceptable derivatives thereof, with a pharmaceutically acceptable glidant, resulting in a mixture characterized by a pharmaceutically acceptable measure of homogeneity.

Another object of the present Invention is to provide a pharmaceutical formulation comprising zidovudine, or a pharmaceutically acceptable derivative thereof, and lamivudine, or a pharmaceutically acceptable derivative thereof, together with one or more pharmaceutically acceptable carriers and optionally, other therapeutic and/or prophylactic ingredients. The carrier(s) must be pharmaceutically acceptable in the sense of being compatible with the other ingredients of the formulation and not deleterious to the recipient thereof.

In a first aspect, the present Invention provides a pharmaceutical composition, comprising:
a) a safe and therapeutically effective amount of lamivudine or a pharmaceutically acceptable derivative thereof;
b) a safe and therapeutically effective amount of zidovudine or a pharmaceutically acceptable derivative thereof; and
c) a pharmaceutically acceptable glidant;
wherein the pharmaceutically acceptable glidant is present in an amount of 0.05% to 10.0% by weight.

The phrase "safe and therapeutically effective amount," as used herein, means a sufficient amount of a drug, compound, composition, product or pharmaceutical agent to abate or reverse or treat a malady in a human or other mammal without severely harming the tissues of the mammal to which the drug or pharmaceutical agent is administered.

The phrase "pharmaceutically acceptable derivative," as used herein, means any pharmaceutically acceptable salt, solvate, ester, or salt of such ester, or any other compound which, upon administration to the recipient, is capable of providing (directly or indirectly) the intended active ingredient or any active metabolite or residue thereof.

The phrase "safe and effective amount," as used herein is that amount of an agent that is required to perform the function being sought by the researcher or clinician without severely harming the tissues of the mammal to which the agent is administered.

In a second or alternative aspect, the present invention provides the use of a composition according to the invention in the manufacture of a medicament for use in treating, reversing, reducing or inhibiting retroviral infections in particular HIV infections, in a mammal, in particular a human.

In a further or alternative aspect, the present invention provides the combined use of lamivudine, or a pharmaceutically acceptable derivative thereof, zidovudine, or a pharmaceutically acceptable derivative thereof, and a pharmaceutically acceptable glidant in the manufacture of a medicament for the treatment of a retroviral infection, in particular an HIV infection; wherein the pharmaceutically acceptable glidant is present in an amount of 0.05% to 10.0% by weight.

It will be appreciated by those skilled in the art that reference herein to "treatment" extends to both the prophylaxis and the treatment of an established malady, infection or its symptoms.

The compositions of the present Invention employ a safe and therapeutically effective amount of 3'-azido-3'-deoxythymidine (zidovudine) and its pharmaceutically acceptable salts, solvates and derivatives thereof, a safe and therapeutically effective amount (-)2',3'-dideoxy,3'-thyacytidine (lamivudine) and its pharmaceutically acceptable salts, solvates and derivatives thereof, along with a safe and effective amount of 0.05% to 10.0% by weight of a pharmaceutically acceptable glidant to maintain the compositions homogeneity prior to tablet compression. Typically the particle size of the two active ingredients will be different. The pharmaceutical formulation is homogenous in the sense that the active ingredients are substantially evenly dispersed throughout that part of the finished formulation which includes lamivudine, zidovudine and the glidant. For example, in the case of a film coated compression tablet, the active ingredients are evenly dispersed through the tablet core.

The compositions of the present invention may optionally employ a safe and effective amount of a diluent, a safe and effective amount of a disintegrant, and a safe and effective amount of a lubricant or any other safe and effective amounts of excipients commonly used in the art.

Lamivudine (also known as 3TC™), chemically known as (2R,cis)-4-amino-1-(2-hydroxymethyl-1,3-oxathiolan-5-yl)-(1H)-pyrimidin-2-one and also know as (-)-2',3'-dideoxy, 3'thyacytidine, is a synthetic nucleoside analogue with antiviral activity against human immunodeficiency virus (HIV).

*In vitro* studies have demonstrated that, intracellularly, lamivudine is phosphorilated to its active 5'-triphosphate metabolite (L-TP). The principle mode of action of L-TP is inhibition of reverse transcription via viral DNA chain termination. L-TP also inhibits the RNA- and DNA-dependent DNA polymerase activities of retroviral reverse transcriptase.

Enantiomers of 2',3'-dideoxy, 3'thyacytidine are equipotent against HIV; however, the (-)-enantiomer (lamivudine) has considerably lower cytotoxicity than the (+)-enantiomer. Particularly, the (-)-enantiomer (lamivudine), (-)2',3'-dideoxy, 3'thyacytidine, is provided substantially free of the (+)-enantiomer, that is to say than about 10% w/w of the(+)-enantiomer, particularly no more than about 5%, and more particularly less than about 1% w/w is present. Methods for the preparation of lamivudine are described in, inter alia, WO 92/20669 and WO 95/29174.

The phrase "pharmaceutically acceptable derivative of lamivudine" as used herein, means any pharmaceutically acceptable salt, solvate, ester, or salt of such ester, of lamivudine, or any other compound which, upon administration to the recipient, is capable of providing (directly or indirectly) lamivudine or any antivirally active metabolite or residue thereof.

Zidovudine, chemically known as 3'-azido-3'-deoxythymidine, is a pyrimidine nucleoside analogue. Intracellularly, zidovudine is enzymatically converted to zidovudine triphosphate. Zidovudine triphosphate interferes with the HIV viral RNA dependent DNA polymerase (reverse transcriptase) and thus, inhibits viral replication.

The phrase "pharmaceutically acceptable derivative of zidovudine" as used herein means any pharmaceutically acceptable salt, solvate, ester, or salt of such ester, of zidovudine, or any other compound which, upon administration to the recipient, is capable of providing (directly or indirectly) zidovudine or any antivirally active metabolite or residue thereof. Methods for the preparation of zidovudine are described in United States. Patent No. 5,011,829.

Glidants are substances which have traditionally been used to improve the flow characteristics of granulations and powders by reducing interparticulate friction. See, Remington, The Science & Practice of Pharmacy, p. 1619, 19th ed. (1995) and see Lieberman, Lachman, & Schwartz, Pharmaceutical Dosage Forms: Tablets, Volume 1, p. 177-178 (1989). Improving flow characteristics helps to reduce tablet press clogging and malfunction and minimizes tablet weight variation. Glidants are typically added to pharmaceutical compositions just prior to tablet compression to facilitate the flow of granular material into the die cavities of tablet presses. A commonly used glidant is silicon dioxide (SiO₂), also referred to as colloidal silica, fumed silicon dioxide, fumed silica, light anhydrous silicic acid, or silicic anhydride. Silicon dioxide is sold under the tradenames AEROSIL™ and CAB-O-SIL™. Other glidants include asbestos free talc, sodium aluminosilicate, calcium silicate, powdered cellulose, microcrystalline cellulose, corn starch, sodium benzoate, calcium carbonate, magnesium carbonate, metallic stearates, calcium stearate, magnesium stearate, zinc stearate, stearowet C, starch, starch 1500, magnesium lauryl sulfate, or magnesium oxide.

The ability of glidants to improve flow characteristics depends on:
(i) their chemical characteristics in relation to the chemical characteristics of the other ingredients of the composition, and;
(ii) physical characteristics such as the size, shape, and distribution, of the glidants and the other components of the granulation or powder composition, as well as the moisture content and temperature of the composition.

Investigation into the problem of active ingredient segregation in pharmaceutical compositions, powders and granulates has led the present Inventors to the surprising discovery that glidants may be used to reduce segregation of active ingredients and thus improve the homogeneity of pharmaceutical compositions, powders and granulates. The present Invention employs from 0.05% to 10.0% glidant. Below about 0.05% the homogeneity may not be sufficient and with amounts greater that 10.0% no additional homogeneity is gained.

Silicon dioxide is a preferred glidant because it is relatively inert. A preferred form of silicon dioxide is fumed colloidal silicon dioxide, which is submicroscopic fumed silica. It is a light, non-gritty amorphous powder. Particularly 0.05% to 1.0% colloidal silicon dioxide is used because below about 0.05% the homogeneity may not be sufficient and with amounts greater that 1.0% no additional homogeneity is gained.

Where glidants are used to improve flow characteristics, they are typically added to the composition immediately prior to compression during the lubrication step. See, Remington, The Science & Practice of Pharmacy, p. 1619, 19th ed. (1995). However, the present Invention makes use of glidants in the initial mixture to improve and maintain homogeneity during handling prior to compression.

The Invention is preferably presented as a pharmaceutical formulation suitable for oral administration. Such formulations may conveniently be presented as discrete units such as tablets, caplets, or any other form suitable for oral administration and compatible with the compositions of the present Invention, each containing a predetermined amount of the active ingredients. A particularly suitable formulation is a dry compression tablet. Such formulations may contain safe and effective amounts of conventional excipients such as binding agents, fillers, lubricants, or disintegrants. The tablets may also be coated according to any method known to persons skilled in the art that would not interfere with the tablets release properties, or the other physical or chemical characteristics of the present Invention. Tablet coating is further described and delineated by Remington, The Science & Practice of Pharmacy 19th ed. 1995. When desired, the above formulations may also be modified by any method known to persons skilled in the art to achieve sustained release of active ingredients. The formulations may also include a safe and effective amount of other active ingredients, such as antimicrobial agents or preservatives.

These compositions of the present Invention are suitable for administration to humans or other mammals particularly via an oral route of administration. However, other routes as utilized by medical practitioners and others skilled in the art of pharmaceutical dosage administration such as Pharmacists and Nurses are not foreclosed. One such method would be the crushing of a solid dosage form, mixing with a suitable administration vehicle and administering rectally as an enema. Other routes of administration might include: topical and inhalation.

It will be appreciated by those skilled in the art that the amount of active ingredients required for use in treatment will vary according to a variety of factors, including the nature of the condition being treated and the age and condition of the patient, and will ultimately be at the discretion of the attending physician, veterinarian or health care practitioner. In general, however, the current recommended oral dose of lamivudine for adults and adolescents is 150mg twice daily administered in combination with zidovudine. For adults with low body weights (less than 50 kg or 110 lb.) the current recommended oral dose of lamivudine is 2mg/kg twice daily administered in combination with zidovudine. The recommended oral dose of lamivudine in pediatric patients 3 months to 12 years of age is 4mg/kg twice daily, up to a maximum of 150mg twice daily administered in combination with zidovudine.

In general, the current recommended oral dose of zidovudine is 600mg per day in divided doses in combination with other antiretroviral agents. The recommended oral dose in pediatric patents 3 months to 12 years of age is 180mg/m² every 6 hours or 720mg/m² per day not to exceed 200mg every 6 hours.

Compositions of the present Invention enable patients greater freedom from multiple dosage medication regimens and ease the needed diligence required in remembering complex daily dosing times and schedules. By combining lamivudine and zidovudine in to a single dosage form, the desired daily doses may be presented in a single dose or as divided doses, particularly as divided doses, administered at appropriate intervals, for example as two, three, four or more sub-doses per day, particularly as two sub-doses per day.

The compositions of the present Invention conveniently allow administration of two separate compounds in unit dosage form containing, for example, from 15 to 1000mg of lamivudine, particularly from 100 to 500mg of lamivudine and most particularly 150mg of lamivudine, and from 30 to 1000mg of zidovudine, particularly from 200mg to 500mg zidovudine and most particularly 300mg of zidovudine per unit dosage form.

The composition of the present Invention may be used in combination with other pharmaceutical formulations as a component of a multiple drug treatment regimen.

Compositions of the present Invention may also be packaged as articles of manufacture comprising a safe and therapeutically effective amount of lamivudine, or a pharmaceutically acceptable derivative thereof; a safe and therapeutically effective amount of zidovudine, or a pharmaceutically acceptable derivative thereof and a safe and effective amount of 0.05% to 10.0% by weight of a pharmaceutically acceptable glidant.

Any of the various methods known by persons skilled in the art for packaging tablets, caplets, or other solid dosage forms suitable for oral administration, that will not degrade the components of the present Invention, are suitable for use in packaging. Tablets, caplets, or other solid dosage forms suitable for oral administration, may be packaged and contained in various packaging materials particularly glass and plastic bottles and also including unit dose blister packaging. The packaging material may also have labeling and information related to the pharmaceutical composition printed thereon. Additionally, an article of manufacture may contain a brochure, report, notice, pamphlet, or leaflet containing product information. This form of pharmaceutical information is referred to in the pharmaceutical industry as a "package insert." A package insert may be attached to or included with a pharmaceutical article of manufacture. The package insert and any article of manufacture labeling provides information relating to the pharmaceutical composition. The information and labeling provides various forms of information utilized by healthcare professionals and patients, describing the composition, its dosage and various other parameters required by regulatory agencies such as the United States Food and Drug Agencies.

The compositions of the present Invention can be formulated using methods and techniques suitable for the compositions physical and chemical characteristics and that are commonly employed by persons skilled in the art in preparing oral dosage forms utilizing a dry press granulation. Remington, The Science & Practice of Pharmacy, p. 1615-1623, 1625-1648, and other applicable sections, 19th ed. (1995).

Compositions of the present Invention can be administered to a human or other mammal in a safe and effective amount as described herein. These safe and effective amounts will vary according to the type and size of mammal being treated and the desired results of the treatment.

### EXAMPLES

The following examples further describe and demonstrate particular embodiments within the scope of the present Invention. The examples are given solely for illustration.

### Example I

| Ingredients | Amount (mg) |
|---|---|
| zidovudine | 300.00 |
| lamivudine | 150.00 |
| microcrystalline cellulose NF | 269.63 |
| sodium starch glycolate NF | 22.50 |
| colloidal silicone dioxide NF | 2.25 |
| magnesium stearate | 5.63 |

### Example II

### Preparation

The quantities of the present example of manufacturing procedure are based on a typical batch size of 400 kg and may be adjusted depending on batch size.

First, the components are weighed from bulk containers in the following amounts:

| Ingredients | Amount (kg) |
|---|---|
| zidovudine | 160.00 |
| lamivudine | 80.00 |
| microcrystalline cellulose NF | 143.80 |
| sodium starch glycolate NF | 12.00 |
| colloidal silicone dioxide NF | 1.20 |
| magnesium stearate | 3.00 |

The components are then sieved using a Russell-SIV equipped with 14 mesh (1.4mm opening) or an equivalent sieve and mesh, and deposited into a stainless-steel blending container.

The zidovudine, lamivudine, microcrystalline cellulose NF, sodium starch glycolate NF, and colloidal silicone dioxide NF are blended for 20 minutes using a suitable blender, such as a Matcon-Buls bin-type blender, a V-blender or equivalent. The magnesium stearate is then added to the mixture and blending is continued for approximately 2 minutes.

The lubricated blend is then compressed using a suitable rotary tablet press, typically a Courtoy R-190, R-200 or equivalent. In-process controls for tablet weight and hardness are applied at appropriate intervals throughout the compression run and adjustments to the tablet press are made as necessary.

## Claims

1. A pharmaceutical composition comprising:
i) a safe and therapeutically effective amount of lamivudine or a pharmaceutically acceptable derivative thereof;
ii) a safe and therapeutically effective amount of zidovudine or a pharmaceutically acceptable derivative thereof; and
iii) a pharmaceutically acceptable glidant;
wherein the pharmaceutically acceptable glidant is present in an amount of 0.05% to 10.0% by weight.

2. A pharmaceutical composition according to Claim 1, wherein the pharmaceutically acceptable glidant is selected from a group consisting of: silicon dioxide, colloidal silicon dioxide, fumed silicon dioxide, sodium aluminosilicate, calcium silicate, powdered cellulose, microcrystalline cellulose, corn starch, sodium benzoate, calcium carbonate, magnesium carbonate, asbestos free talc, metallic stearates, calcium stearate, magnesium stearate, zinc stearate, stearowet C, starch, starch 1500, magnesium lauryl sulfate, or magnesium oxide.

3. The pharmaceutical composition according to Claim 2 wherein the pharmaceutically acceptable glidant is fumed silicon dioxide.

4. The pharmaceutical composition according to any one of Claims 1 to 3 wherein the amount of lamivudine is from 15 to 1000 mg per unit dosage form.

5. The pharmaceutical composition according to Claim 4 wherein the amount of lamivudine is from 100 to 500 mg per unit dosage form.

6. The pharmaceutical composition according to Claim 5 wherein the amount of lamivudine is 150mg per unit dosage form.

7. The pharmaceutical composition according to any one of Claims 1 to 6 wherein the amount of zidovudine is from 30 to 1000 mg per unit dosage form.

8. The pharmaceutical composition according to Claim 7 wherein the amount of zidovudine is from 200 to 500mg per unit dosage form.

9. The pharmaceutical composition according to Claim 8 wherein the amount of zidovudine is 300mg per unit dosage form.

10. The pharmaceutical composition according to any one of Claims 1 to 9 wherein lamivudine is provided substantially free of the corresponding (+)-enantiomer.

11. The pharmaceutical composition according to any one of Claims 1 to 9 wherein the (+)-enantiomer is present in an amount of not more than 5% w/w of the amount of lamivudine.

12. A pharmaceutical composition according to any one of Claims 1 to 11 wherein the composition is coated with a pharmaceutically acceptable coating.

13. A method for increasing and maintaining the homogeneity of a pharmaceutical composition comprising more that one active ingredient by including a safe and effective amount of a pharmaceutically acceptable glidant.

14. The method according to claim 13 for increasing and maintaining the homogeneity of a pharmaceutical composition comprising more that one active ingredient by including a safe and effective amount of a pharmaceutically acceptable glidant, wherein the glidant is selected from the group consisting of: silicon dioxide, colloidal silicon dioxide, fumed silicon dioxide, sodium aluminosilicate, calcium silicate, powdered cellulose, microcrystalline cellulose, corn starch, sodium benzoate, calcium carbonate, magnesium carbonate, asbestos free talc, metallic stearates, calcium stearate, magnesium stearate, zinc stearate, stearowet C, starch, starch 1500, magnesium lauryl sulfate, or magnesium oxide.

15. The method according to Claim 14 for increasing and maintaining the homogeneity of a pharmaceutical composition comprising more that one active ingredient by including a safe and effective amount of a pharmaceutically acceptable glidant, wherein the glidant is selected from the group consisting of: fumed silicon dioxide, colloidal silicon dioxide, or fumed colloidal silicon dioxide.

16. The use of lamivudine or a pharmaceutically acceptable derivative thereof, zidovudine or a pharmaceutically acceptable derivative thereof and a pharmaceutically acceptable glidant in the manufacture of a medicament of the treatment of a retroviral infection; wherein the pharmaceutically acceptable glidant is present in an amount of 0.05% to 10.0% by weight.

17. The use according to Claim 16 wherein the the retrovirus is an immunodeficiency virus, including HIV.

18. An article of manufacture comprising:
i) packaging material; and
ii) a pharmaceutical composition contained within the packaging material, comprising:
a) a safe and therapeutically effective amount of lamivudine or a pharmaceutically acceptable derivative thereof;
b) a safe and therapeutically effective amount of zidovudine or a pharmaceutically acceptable derivative thereof; and
c) a pharmaceutically acceptable glidant;
wherein the pharmaceutically acceptable glidant is present in an amount of 0.05% to 10.0% by weight.

19. An article of manufacture of Claim 18 additionally comprising a brochure containing product information.

20. An article of manufacture according to Claim 18 or Claim 19 wherein the packaging material is unit dose blister packaging.

21. A process for the preparation of a pharmaceutical composition as claimed in any of claims 1-12 which process comprises admixture of lamivudine or a pharmaceutically acceptable derivative thereof, zidovudine or a pharmaceutically acceptable derivative thereof and a pharmaceutically acceptable glidant.

## Patentansprüche

1. Pharmazeutische Zusammensetzung, die:
i) eine sichere und therapeutisch wirksame Menge von Lamivudin oder einem pharmazeutisch akzeptablen Derivat davon;
ii) eine sichere und therapeutisch wirksame Menge von Zidovudin oder einem pharmazeutisch akzeptablen Derivat davon; und
iii) ein pharmazeutisch akzeptables Fließregulierungsmittel umfaßt:;
worin das pharmazeutisch akzeptable Fließregulierungsmittel in einer Menge von 0,05 bis 10,0 Gew.% vorhanden ist.

2. Pharmazeutische Zusammensetzung gemäß Anspruch 1, worin das pharmazeutisch akzeptable Fließregulierungsmittel aus einer Gruppe ausgewählt ist, die aus: Siliziumdioxid, kolloidalem Siliziumdioxid, hochdispersem ("fumed") Siliziumdioxid, Natriumaluminosilikat, Calciumsilikat, gepulverter Zellulose, mikrokristalliner Zellulose, Maisstärke, Natriumbenzoat, Calciumcarbonat, Magnesiumcarbonat, asbestfreiem Talkum, Metallstearaten, Calciumstearat, Magnesiumstearat, Zinkstearat, Stearowet C, Stärke, Stärke 1500, Magnesiumlaurylsulfat oder Magnesiumoxid besteht.

3. Pharmazeutische Zusammensetzung gemäß Anspruch 2, worin das pharmazeutisch akzeptable Fließregulierungsmittel hochdisperses ("fumed") Siliziumdioxid ist.

4. Pharmazeutische Zusammensetzung gemäß einem der Ansprüche 1 bis 3, worin die Lamivudin-Menge 15 bis 1.000 mg pro Einheitsarzneiform beträgt.

5. Pharmazeutische Zusammensetzung gemäß Anspruch 4, worin die Lamivudin-Menge 100 bis 500 mg pro Einheitsarzneiform beträgt.

6. Pharmazeutische Zusammensetzung gemäß Anspruch 5, worin die Lamivudin-Menge 150 mg pro Einheitsarzneiform beträgt.

7. Pharmazeutische Zusammensetzung gemäß einem der Ansprüche 1 bis 6, worin die Zidovudin-Menge 30 bis 1.000 mg pro Einheitsarzneiform beträgt.

8. Pharmazeutische Zusammensetzung gemäß Anspruch 7, worin die Zidovudin-Menge 200 bis 500 mg pro Einheitsarzneiform beträgt.

9. Pharmazeutische Zusammensetzung gemäß Anspruch 8, worin die Zidovudin-Menge 300 mg pro Einheitsarzneiform beträgt.

10. Pharmazeutische Zusammensetzung gemäß einem der Ansprüche 1 bis 9, worin Lamivudin im wesentlichen frei vom entsprechenden (+)-Enantiomer bereitgestellt ist.

11. Pharmazeutische Zusammensetzung gemäß einem der Ansprüche 1 bis 9, worin das (+)-Enantiomer in einer Menge von nicht mehr als 5 % G/G der Lamivudin-Menge vorhanden ist.

12. Pharmazeutische Zusammensetzung gemäß einem der Ansprüche 1 bis 11, worin die Zusammensetzung mit einem pharmazeutisch akzeptablen Überzug umhüllt ist.

13. Verfahren zur Erhöhung und Aufrechterhaltung der Homogenität einer pharmazeutischen Zusammensetzung, die mehr als einen Wirkstoff umfaßt, durch Einschließen einer sicheren und wirksamen Menge eines pharmazeutisch akzeptablen Fließregulierungsmittels.

14. Verfahren gemäß Anspruch 13 zur Erhöhung und Aufrechterhaltung der Homogenität einer pharmazeutischen Zusammensetzung, die mehr als einen Wirkstoff umfaßt, durch Einschließen einer sicheren und wirksamen Menge eines pharmazeutisch akzeptablen Fließregulierungsmittels, worin das Fließregulierungsmittel aus der Gruppe ausgewählt ist, die aus: Siliziumdioxid, kolloidalem Siliziumdioxid, hochdispersem ("fumed") Siliziumdioxid, Natriumaluminosilikat, Calciumsilikat, gepulverter Zellulose, mikrokristalliner Zellulose, Maisstärke, Natriumbenzoat, Calciumcarbonat, Magnesiumcarbonat, asbestfreiem Talkum, Metallstearaten, Calciumstearat, Magnesiumstearat, Zinkstearat, Stearowet C, Stärke, Stärke 1500, Magnesiumlaurylsulfat oder Magnesiumoxid besteht.

15. Verfahren gemäß Anspruch 14 zur Erhöhung und Aufrechterhaltung der Homogenität einer pharmazeutischen Zusammensetzung, die mehr als einen Wirkstoff umfaßt, durch Einschließen einer sicheren und wirksamen Menge eines pharmazeutisch akzeptablen Fließregulierungsmittels, worin das Fließregulierungsmittel aus der Gruppe ausgewählt ist, die aus: hochdispersem Siliziumdioxid, kolloidalem Siliziumdioxid oder hochdispersem kolloidalem Siliziumdioxid besteht.

16. Verwendung von Lamivudin oder einem pharmazeutisch akzeptablen Derivat davon, Zidovudin oder einem pharmazeutisch akzeptablen Derivat davon und einem pharmazeutisch akzeptablen Fließregulierungsmittel in der Herstellung eines Medikaments zur Behandlung einer Retrovirusinfektion, worin das pharmazeutisch akzeptable Fließregulierungsmittel in einer Menge von 0,05 bis 10,0 Gew.% vorhanden ist.

17. Verwendung gemäß Anspruch 16, worin das Retrovirus ein Immundefizienzvirus ist, einschließlich HIV.

18. Herstellungsgegenstand, umfassend:
i) Verpackungsmaterial; und
ii) eine pharmazeutische Zusammensetzung, die im Verpackungsmaterial enthalten ist, umfassend:
a) eine sichere und therapeutisch wirksame Menge von Lamivudin oder einem pharmazeutisch akzeptablen Derivat davon;
b) eine sichere und therapeutisch wirksame Menge von Zidovudin oder einem pharmazeutisch akzeptablen Derivat davon; und
c) ein pharmazeutisch akzeptables Fließregulierungsmittel;
worin das pharmazeutisch akzeptable Fließregulierungsmittel in einer Menge von 0,05 bis 10,0 Gew.% vorhanden ist.

19. Herstellungsgegenstand gemäß Anspruch 18, der zusätzlich eine Broschüre umfaßt, die Produktinformationen enthält.

20. Herstellungsgegenstand gemäß Anspruch 18 oder 19, worin das Verpackungsmaterial eine Einheitsdosis-Blisterverpackung ist.

21. Verfahren zur Herstellung einer pharmazeutischen Zusammensetzung gemäß einem der Ansprüche 1 bis 12, wobei das Verfahren das Vermischen von Lamivudin oder einem pharmazeutisch akzeptablen Derivat davon, Zidovudin oder einem pharmazeutisch akzeptablen Derivat davon und einem pharmazeutisch akzeptablen Fließregulierungsmittel umfaßt.

## Revendications

1. Composition pharmaceutique comprenant :
i) une quantité sans risque et efficace du point de vue thérapeutique de lamivudine ou d'un dérivé de celle-ci acceptable du point de vue pharmaceutique,
ii) une quantité sans risque et efficace du point de vue thérapeutique de zidovudine ou d'un dérivé de celle-ci acceptable du point de vue pharmaceutique, et
iii) un agent de glissement acceptable du point de vue pharmaceutique ;
dans laquelle l'agent de glissement acceptable du point de vue pharmaceutique est présent en une quantité de 0,05 % à 10,0 % du poids.

2. Composition pharmaceutique selon la revendication 1, dans laquelle l'agent de glissement acceptable du point de vue pharmaceutique est choisi dans un groupe consistant en : dioxyde de silicium, dioxyde colloïdal de silicium, dioxyde fumé de silicium, silicate alumineux de sodium, silicate de calcium, cellulose pulvérisée, cellulose microcristalline, amidon de blé, benzoate de sodium, carbonate de calcium, carbonate de magnésium, talc sans amiante, stéarates métalliques, stéarate de calcium, stéarate de magnésium, stéarate de zinc, stéarowet C, amidon, amidon 1500, lauryl sulfate de magnésium ou oxyde de magnésium.

3. Composition pharmaceutique selon la revendication 2, dans laquelle l'agent de glissement acceptable du point de vue pharmaceutique est du dioxyde fumé de silicium.

4. Composition pharmaceutique selon l'une quelconque des revendications 1 à 3, dans laquelle la quantité de lamivudine est de 15 à 1000 mg par forme unitaire de dosage.

5. Composition pharmaceutique selon la revendication 4, dans laquelle la quantité de lamivudine est de 100 à 500 mg par forme unitaire de dosage.

6. Composition pharmaceutique selon la revendication 5, dans laquelle la quantité de lamivudine est de 150 mg par forme unitaire de dosage.

7. Composition pharmaceutique selon l'une quelconque des revendications 1 à 6, dans laquelle la quantité de zidovudine est de 30 à 1000 mg par forme unitaire de dosage.

8. Composition pharmaceutique selon la revendication 7, dans laquelle la quantité de zidovudine est de 200 à 500 mg par forme unitaire de dosage.

9. Composition pharmaceutique selon la revendication 8, dans laquelle la quantité de zidovudine est de 300 mg par forme unitaire de dosage.

10. Composition pharmaceutique selon l'une quelconque des revendications 1 à 9, dans laquelle la lamivudine est prévue en substance exempte de l'énantiomère (+)- correspondant.

11. Composition pharmaceutique selon l'une quelconque des revendications 1 à 9, dans laquelle l'énantiomère (+)- est présent en une quantité de pas plus de 5 % du poids de la quantité de lamivudine.

12. Composition pharmaceutique selon l'une quelconque des revendications 1 à 11, dans laquelle la composition est enrobée d'un enrobage acceptable du point de vue pharmaceutique.

13. Procédé pour accroître et maintenir l'homogénéité d'une composition pharmaceutique comprenant plus d'un ingrédient actif en incluant une quantité sans risque et efficace d'un agent de glissement acceptable du point de vue pharmaceutique.

14. Procédé selon la revendication 13 pour accroître et maintenir l'homogénéité d'une composition pharmaceutique comprenant plus d'un ingrédient actif en incluant une quantité sans risque et efficace d'un agent de glissement acceptable du point de vue pharmaceutique, dans lequel l'agent de glissement est choisi dans le groupe consistant en : dioxyde de silicium, dioxyde colloïdal de silicium, dioxyde fumé de silicium, silicate alumineux de sodium, silicate de calcium, cellulose pulvérisée, cellulose microcristalline, amidon de blé, benzoate de sodium, carbonate de calcium, carbonate de magnésium, talc sans amiante, stéarates métalliques, stéarate de calcium, stéarate de magnésium, stéarate de zinc, stéarowet C, amidon, amidon 1500, lauryl sulfate de magnésium ou oxyde de magnésium.

15. Procédé selon la revendication 14 pour accroître et maintenir l'homogénéité d'une composition pharmaceutique comprenant plus d'un ingrédient actif en incluant une quantité sans risque et efficace d'un agent de glissement acceptable du point de vue pharmaceutique, dans lequel l'agent de glissement est choisi dans le groupe consistant en : dioxyde fumé de silicium, dioxyde colloïdal de silicium, ou dioxyde colloïdal fumé de silicium.

16. Utilisation de lamivudine ou d'un dérivé de celle-ci acceptable du point de vue pharmaceutique, de zidovudine ou d'un dérivé de celle-ci acceptable du point de vue pharmaceutique et d'un agent de glissement acceptable du point de vue pharmaceutique dans la fabrication d'un médicament du traitement d'une infection rétrovirale, dans laquelle l'agent de glissement acceptable du point de vue pharmaceutique est présent en une quantité de 0,05 % à 10,0 % du poids.

17. Utilisation selon la revendication 16, dans laquelle le rétrovirus est un virus de l'immunodéficience, notamment le HIV.

18. Article de fabrication comprenant :
i) du matériel d'emballage et
ii) une composition pharmaceutique contenue dans le matériel d'emballage, comprenant :
a) une quantité sans risque et efficace du point de vue thérapeutique de lamivudine ou d'un dérivé de celle-ci acceptable du point de vue pharmaceutique,
b) une quantité sans risque et efficace du point de vue thérapeutique de zidovudine ou d'un dérivé de celle-ci acceptable du point de vue pharmaceutique,
c) un agent de glissement acceptable du point de vue pharmaceutique,
dans lequel l'agent de glissement acceptable du point de vue pharmaceutique est présent en une quantité de 0,05 % à 10,0 % du poids.

19. Article de fabrication selon la revendication 18, comprenant en plus une brochure contenant des informations sur le produit.

20. Article de fabrication selon la revendication 18 ou la revendication 19, dans lequel le matériel d'emballage est un emballage d'unidose sous blister.

21. Procédé de préparation d'une composition pharmaceutique selon l'une quelconque des revendications 1-12, procédé qui comprend l'addition de lamivudine ou d'un dérivé de celle-ci acceptable du point de vue pharmaceutique, de zidovudine ou d'un dérivé de celle-ci acceptable du point de vue pharmaceutique et d'un agent de glissement acceptable du point de vue pharmaceutique.
